Europäisches Patentamt

**European Patent Office**

**Office européen des brevets**

(11) Publication number: **0 142 864**
**A2**

(12) **EUROPEAN PATENT APPLICATION**

(21) Application number: **84114043.7**

(22) Date of filing: **20.11.84**

(51) Int. Cl.⁴: **A 61 B 6/00**
**A 61 B 6/06, H 04 N 5/32**

(30) Priority: **22.11.83 JP 220123/83**

(43) Date of publication of application:
**29.05.85 · Bulletin · 85/22**

(84) Designated Contracting States:
**DE FR GB NL**

(71) Applicant: **Kabushiki Kaisha Toshiba**
**72, Horikawa-cho Saiwai-ku**
**Kawasaki-shi Kanagawa-ken 210(JP)**

(72) Inventor: **Kikuchi, Katsuya c/o Patent Division**
**KABUSHIKI KAISHA TOSHIBA 1-1 Shibaura 1-chome**
**Minato-ku Tokyo 105(JP)**

(72) Inventor: **Fujii, Senzo c/o Patent Division**
**KABUSHIKI KAISHA TOSHIBA 1-1 Shibaura 1-chome**
**Minato-ku Tokyo 105(JP)**

(74) Representative: **Patentanwälte Henkel, Pfenning, Feiler,**
**Hänzel & Meinig**
**Möhlstrasse 37**
**D-8000 München 80(DE)**

(54) **X-ray diagnostic apparatus utilizing x-ray shield member.**

(57) In an X-ray diagnostic apparatus (100), it is very desirable to mittigate the adverse effect of the scattered X-ray components. When the X-ray shield member (50) is positioned in the X-ray projection area (38) during the first X-ray projection period, the first X-ray transmission image data is obtained. From this X-ray transmission image data, the scattered X-ray component data (Isc) is calculated by using the linear interpolation method. When the X-ray shield member (50) is removed from the X-ray projection area 38 during the second X-ray projection period, the second X-ray transmission image data is obtained that includes not only the primary X-ray components but also the scattered X-ray components. The desirable X-ray transmission image data is obtained by subtracting the first image data from the second image data.

./...

# F I G. 4

- 1 -

X-ray diagnostic apparatus
utilizing X-ray shield member

This invention generally relates to an X-ray diagnostic apparatus in which a transmitted X-ray image of an object to be examined, e.g., a patient, is available for diagnostic purposes, and more particularly, to an X-ray diagnostic apparatus by which visible X-ray images of the object can be obtained, based only upon primary X-rays, without any adverse influences caused by the scattered X-rays.

Generally, in the X-ray diagnostic apparatus set forth in the preamble, X-rays incident on an X-ray detector through the object such as a patient contain not only primary X-rays but also X-rays which are scattered by the object under examination. The scattered X-rays constitute one of the major causes of deteriorated contrast and resolution in the transmitted X-ray image. This makes it necessary to eliminate an image component on the scattered X-rays from the transmitted X-ray image data as sensed and provided by the detector.

One of the approaches to eliminate the scattered X-ray component is to use a so-called "Buckey Blade" or an elimination grid for the scattered X-rays (referred to as a "grid"). This approach newly involves a problem in that there is a limit in the scattered X-ray elimination, because the grid per se scattered the X-rays incident thereupon.

The elimination of the scattered X-rays is very significant in the field of X-ray diagnosis for the reasons that it improves an image quality, such as contrast and resolution, and thus allows a logarithm conversion of primary X-rays image data, thereby obtaining an accurate attenuation quantity of X-rays caused when the X-rays pass through the object. Many studies have been made on the scattered X-rays, aiming at their effective elimination. The complicated phenomena of the scattered X-rays impede or almost reject a theoretical approach to this theme. This is the present stage of technology in this field.

For the above background reasons, an object of the present invention is to provide, by introducing a novel technical idea, an X-ray diagnositic apparatus which can effectively eliminate the scattered X-ray image component from the transmitted X-ray image components as obtained by the X-ray detector.

The object of the present invention may be accomplished by providing an X-ray diagnostic apparatus comprising:

an X-ray source for successively generating X-rays;

an X-ray detector for detecting an X-ray image of an object under examination by projecting the X-rays from the X-ray source toward the object, and for converting the detected image into X-ray transmission signals;

an analogue-to-digital converter for converting the X-ray transmission signals into corresponding digital transmission data;

an X-ray shield member having a plurality of X-ray shield materials, for partially blocking the penetration of the X-rays over an X-ray projection area defined by projecting the X-rays from the X-ray source to the X-ray detection means through the object;

a first memory for storing at least first X-ray transmission data acquired during a first X-ray projection period under the condition that the X-ray shield member is inserted into the X-ray projection area;

a first arithmetic operation device for obtaining data representing an intensity distribution of scattered X-ray components, based upon the first transmission data over the entire X-ray projection area by way of an interpolation method;

a second memory for storing at least second transmission data acquired during a second X-ray projection period under the condition that the X-ray shield member is removed from the X-ray projection area; and

a second arithmetic operation device for subtracting said data representing the intensity distribution of scattered X-ray components from the second transmission data, thereby obtaining X-ray transmission data having no adverse influence by the scattered X-ray components.

The particular advantage is obtained in that since the X-ray transmission image having no adverse influence caused by the scattered X-ray components, can be displayed, the resultant image of the object has better contrast and resolution. As a result, such an X-ray diagnostic apparatus according to the invention can provide a precise and excellent diagnosis.

This and other objects and features of the present invention may be best understood by reference to the specification and the accompanying drawings, in which;

Fig. 1 is an illustration for explaining an occurrence of scattered X-rays when an X-ray is projected toward an object under examination;

Fig. 2 shows a graphic representation on an X-ray intensity vs. a detection position on an X-ray detector;

Figs 3A, 3B and 3C graphically illustrate a spatial distribution of the scattered X-rays' intensity;

Fig. 4 shows a schematic block diagram of an X-ray diagnostic apparatus according to one preferred embodiment;

Fig. 5 schematically shows a front view of an X-ray shield member;

Fig. 6 is an illustration for explaining related positions of the X-ray source-to-the detector;

Fig. 7 is a graphic diagram of the relation between the X-ray intensity and the X-ray projection area;

Fig. 8 is an image pattern for explaining the linear interpolation method;

Fig. 9 is a graphic diagram of the relation between the X-ray intensity and the pixel of the detector; and

Fig. 10 is an illustration for explaining in detail an example of the linear interpolation method.

Before proceeding with the various types of preferred embodiments according to the present invention, the principle of the present invention will now be described in detail.

First, a description is made of a phenomenon of the scattered X-ray.

It is assumed that X-rays incident on the object such as a patient, under examination are generally classified into primary X-rays which directly transmit through the object and enter into an X-ray detector, and X-rays absorbed or scattered by the object through interactions of the X-rays with atoms constituting the object. Those scattered ones are called "scattered X-rays". In the energy range of medical X-rays (radiated under 50 KVp to 120 KVp of the X-ray tube voltage), some causes for the scattered X-rays are known, for example, photoelectric effects, Compton effects, Thomson effects, and the like. These phenomena cooperate to cause the scattered X-rays to have adverse effects on the transmitted X-ray image to be described later. In general, because the scattered X-rays incident on the X-ray detector experience multi-scattering within the object, it is very difficult to exactly grasp an intensity and a spatial spread of an incident X-ray beam. This phenomenon is explained as follows.

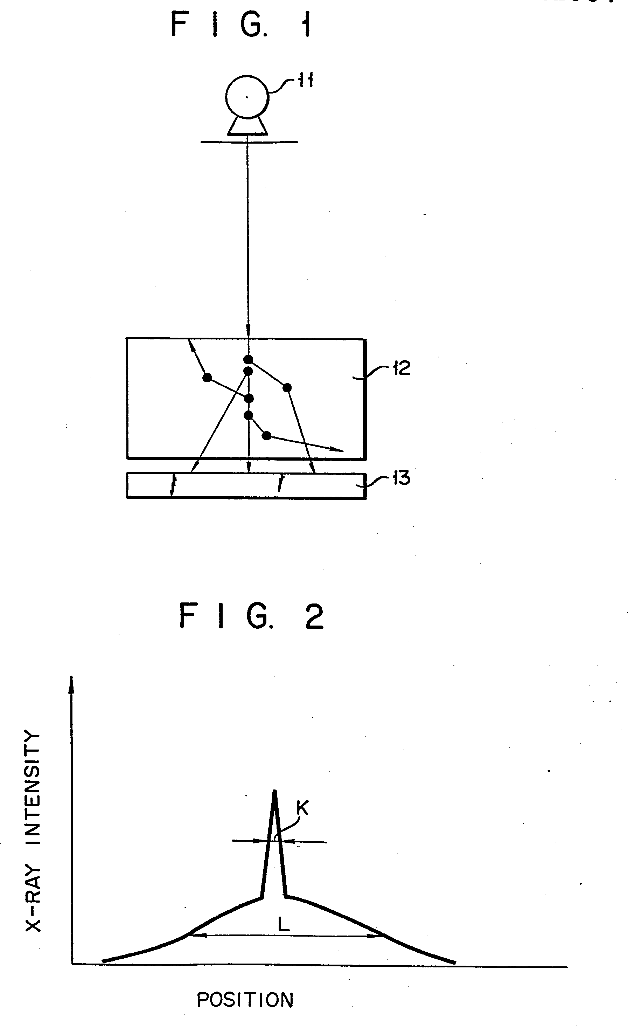

Fig. 1 schematically illustrates how an X-ray radiated from an X-ray source 11, such as an X-ray tube, is scattered within an object 12 under examination and reaches an X-ray detector 13, while depicting a spatial spread with respect to detecting positions of the X-ray detector. Fig. 2 illustrates an X-ray intensity distribution over the detecting positions of the X-ray detector. Fig. 2 illustrates an X-ray intensity distribution over the detecting positions of the X-ray detector 13. As seen from Fig. 2, a narrow spread, or spatial distribution of a sharp peak (as indicated by character K), located substantially at the center of the distribution curve, is caused by an inherent matter of the diagnosis system, for example, an X-ray focal spot and a wide spread (as indicated by character L) is caused by the scattered X-rays.

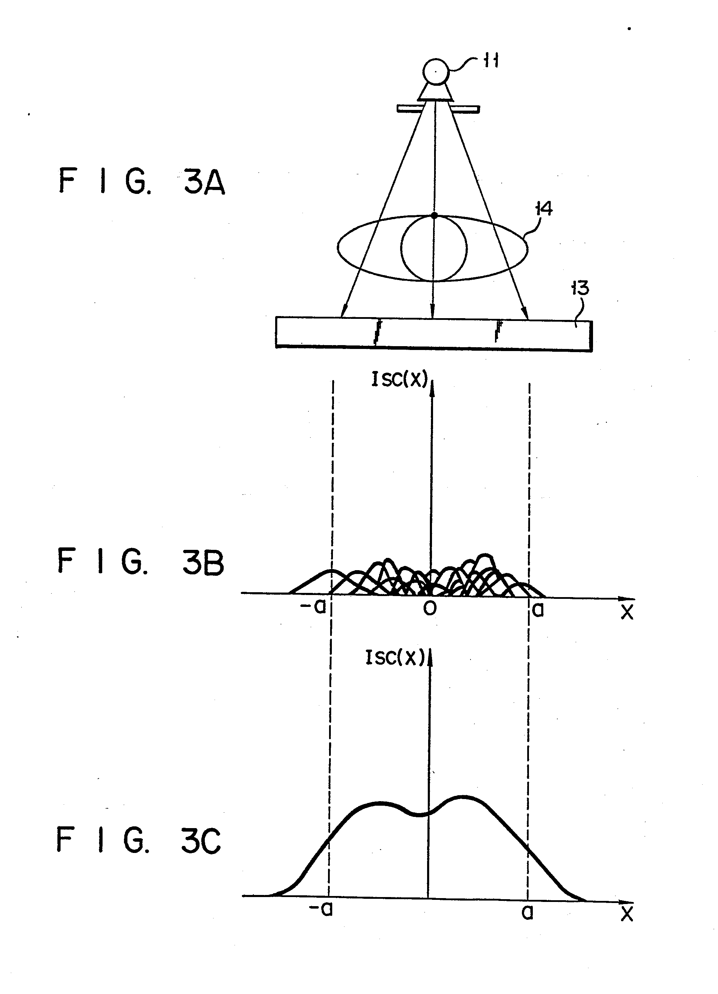

In Fig. 3, a spatial distribution of the scattered X-rays is graphically shown. In Fig. 3A, a narrow X-ray beam is projected toward a body 14. In Fig. 3B, spatial distributions of the respective scattered X-rays are graphically shown. In Fig. 3C, an actual spatial distribution of the scattered X-rays is graphically shown, that is obtained by summing these spatial distributions. The characters "-a" and "a" define an area projected by the X-rays (referred to as an "X-ray projection area") on the detecting positions of the X-ray detector 13. The symbol "Isc(x)" denotes an intensity of the scattered X-rays. For convenience and clarity of illumination, these drawings are illustrated in one dimension.

A total X-ray intensity distribution Im(x, y) incident on the detector is the sum of the primary X-ray intensity distribution Ip(x, y) and the scattered X-ray intensity distribution Ip(x, y) and is given by:

$$Im(x, y) = Ip(X, y) + Isc(x, y) \quad ... \quad (1),$$

where (x, y) indicates coordinates for representing positions on the X-ray detector 13.

As previously described, since the spatial distributions of the scattered X-ray component Isc(x, y) gradually vary over the X-ray projection area, it is practically possible to relatively, precisely guess the scattered X-ray component Isc(x, y) over the X-ray projection area by employing a plurality of the scattered X-ray component data.

The basic of the present invention can be realized based upon the above-described recognition.

In accordance with the present invention, the X-ray is projected toward the object under examination in such a way that an X-ray shield member is interposed between the X-ray source and the X-ray detector within the X-ray projection area. The X-ray projection area is defined by projecting the X-ray from the X-ray source to the X-ray detector through the object. Under such a condition, first transmitted X-ray image data obtained by the detector may contain theoretically the scattered X-ray components only, because the primary X-ray components have been shielded by the X-ray shield member before reaching the X-ray detector. Second transmitted X-ray image data is acquired under the condition that the X-ray shield member is removed from the X-ray projection area. Accordingly, the second image data contains not only the scattered X-ray components but also the primary X-ray components. As a result, subtracting the first image data from the second image data enables desirable image data to be calculated in accordance with the equation 1. This desirable image data involves only the primary X-ray components.

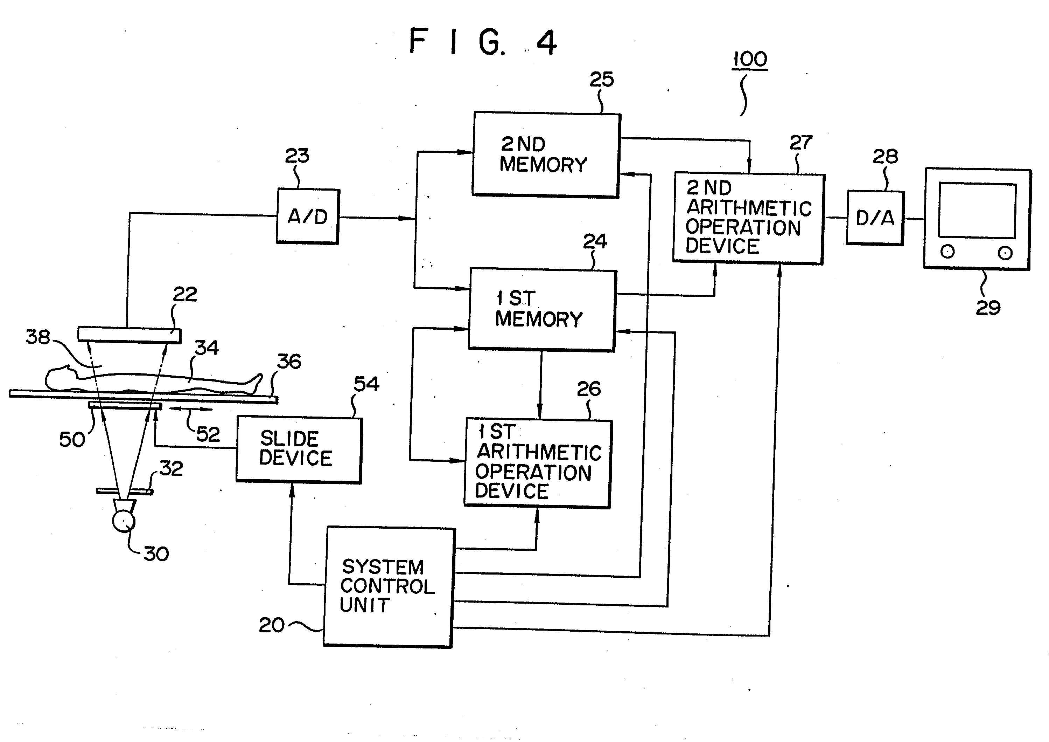

Referring to Fig. 4, a description is made of an X-ray diagnostic apparatus 100 according to the invention, in which the above basic idea is employed.

An X-ray source 30 generates X-rays that are projected through an X-ray diaphragm 32 toward a patient 34 under examination. The patient 32 lies down on a couch 36. The X-ray projection area of the X-ray source 30 is denoted by reference numeral 38, that is defined by projecting the X-ray from the X-ray source 30 to the X-ray detector 22 through the patient 34.

An X-ray shield member 50 is provided under the couch 36. In other words, it is positioned in front of the patient 34 along the X-ray path. This shield member 50 is designed to be slidable in parallel to the patient 34 or the couch 36. The slide operation into the X-ray projection area 38 will be described layer. A slide direction is indicated by an arrow 52.

A system control unit 20 is provided with the X-ray diagnostic apparatus 100. A slide device 54 can mechanically slide the X-ray shield member 50 along the slide direction 52 under the control of the system control unit 20. An X-ray detector 22 is positioned behind the patient 34 along the X-ray path within the X-ray projection area 38. Outputs of the detector 22 are fed to an analogue-to-digital converter (A/D converter) 23. To the A/D converter 23, first and second memories 24 and 25 are connected. The first memory 24 is communicated with a first arithmetic operation device 26 and the second memory 25 is communicated with a second arithmetic operation device 27. These first and second memories 24, 25, and first and second arithmetic operation devices 26, 27 are controlled by the system control unit 20. Outputs of the second arithmetic operation device 27 are fed to a digital-to-analogue converter (D/A converter) 28. The D/A converter 28 is connected to a TV monitor 29.

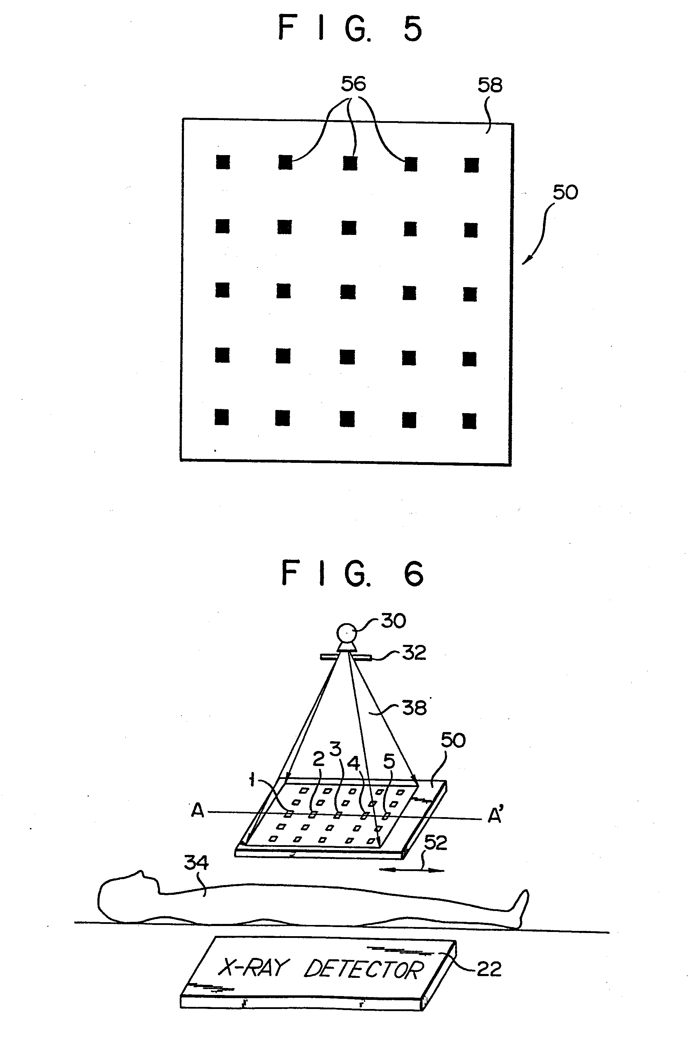

Fig. 5 shows a front view of the X-ray shield member 50. The X-ray shield member 50 is fabricated by a plurality of X-ray shield materials such as lead pieces 56 and a thin plate-like material such as a synthetic resin film 58. These lead pieces 56 are positioned in a matrix in the resin film 58. Each lead pieces 56 has a size of 2mm × 2mm, for example.

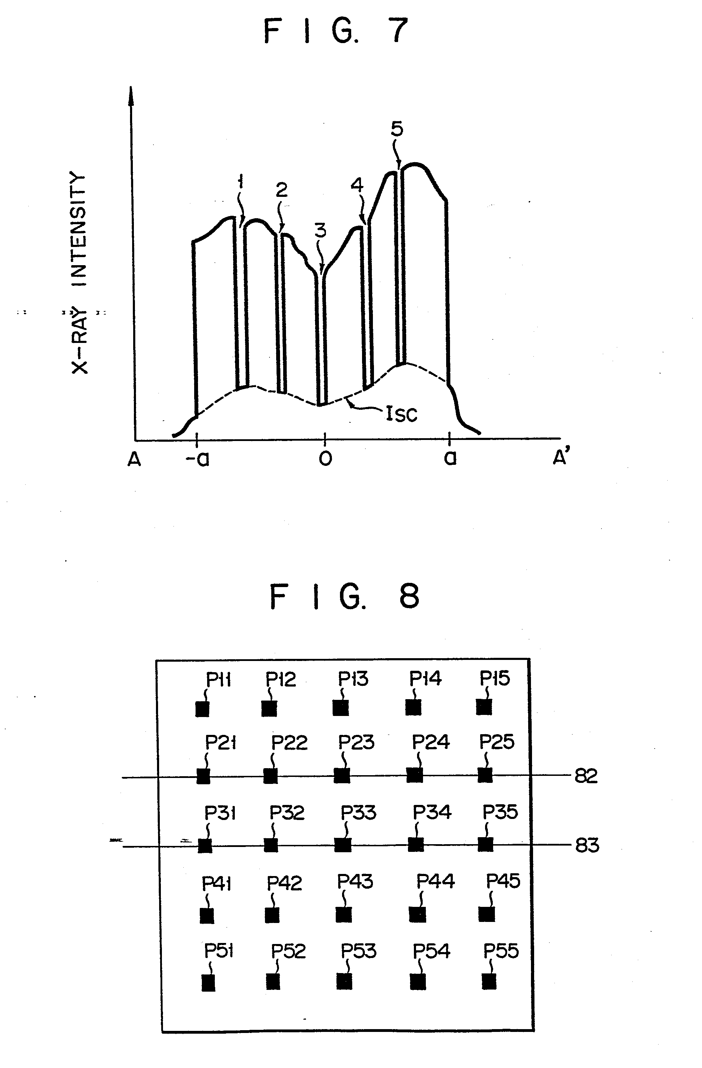

While the X-ray shield member 50 is positioned in the X-ray projection area 38 defined by projecting the X-ray from the X-ray source 30 to the X-ray detector 22 through the object 34, the intensity distribution of the transmitted X-ray image signal is obtained by projecting the X-ray toward the object 34, that is shown by a graphic representation of Fig. 7. This intensity distribution Isc represents one which is taken along the lines A-A' on the surface of the X-ray detector 22. As seen from the distribution curve, the intensity levels of those positions where the lead pieces 56 are positioned (indicated by numerical references 1, 2, 3, 4, and 5) steeply drop. Consequently, these intensity levels indicate the intensity of the scattered X-ray components Isc, because the primary X-ray components are blocked by those lead pieces 56.

It should be noted that the arrangement of the X-ray source 30 and the detector 22 shown in Fig. 6 is reversed, compared with the arrangement shown in Fig. 4.

Operations of the X-ray diagnostic apparatus (100) will now be described with reference to Figs. 4 to 10.

Referring to Fig. 4, the X-ray source 30 is evergized to project the X-ray toward the patient 34 while the X-ray shield member 50 is slid along the longitudinal axis of the couch 36 and positioned within the X-ray projection area 38 by means of the slide device 54. The slide device 54 is controlled by the system control unit 20. The X-ray transmitted through the patient 34 is incident, as the X-ray image upon the X-ray detector 22 such as an image intensifier.

The X-ray image is converted into an analogue X-ray transmission image signal. Thereafter, it is converted by the A/D converter 23 into corresponding digital transmission data. The digital transmission data is temporarily stored as first X-ray transmission data in the first memory 24.

From the digital transmission data stored in the first memory 24, the amount of the scattered X-ray components over the entire pixels can be calculated by the first arithmetic operation device 26 in such a manner that the amount of the scattered X-ray components for the respective pixels is obtained by the internal interpolation method based upon the actual amount of the scattered X-ray components with respect to positions where the lead pieces 56 are present. The resultant data is stored in the first memory 24, that represents the intensity distribution of scattered X-ray components.

Thereafter, another X-ray projection is excuted after the X-ray shield member 50 is completely removed from the X-ray projection area 38. Similarly, X-ray transmission data is temporarily stored as second X-ray transmission data in the second memory 25.

Thereafter, both the calculated data stored in the first memory 24 and the second X-ray transmission data are read from the respective memories 24 and 25 to the second arithmetic operation device 27. The function of this device 27 is as follows. The data representing the intensity distribution of the scattered X-ray components Isc is subtracted from the second X-ray transmission data so as to derive X-ray transmission data containing only the primary X-ray components. In other words, this resultant data excludes the scattered X-ray components.

Then, the finally-obtained transmission data is digital-to-analogue converted in the D/A converter 28, and is displayed in the TV monitor 29. The displayed X-ray image has high image qualities, i.e., better contrast and resolution. Such an advantage of the invention is caused for the following reason. The amount of the scattered X-ray components is calculated in the first arithmetic operation device 26 based upon the first X-ray transmission data that has been taken by partially blocking the penetration of the X-ray by inserting the X-ray shield member 50 into the X-ray projection area 38. The resultant data involves only the intensity distribution of the scattered X-ray components Isc. This data is then subtracted from the second X-ray transmission data that has been taken by extracting the X-ray shield member 50 from the X-ray projection area 38, so that the finally-obtained transmission data contains only the primary X-ray components. Accordingly, the X-ray transmission image displayed on the TV monitor 29 has no adverse influence caused by the scattered X-ray components.

A description will now be made of the linear interpolation method, as the above internal interpolation method, to be introduced into the X-ray diagnostic apparatus 100.

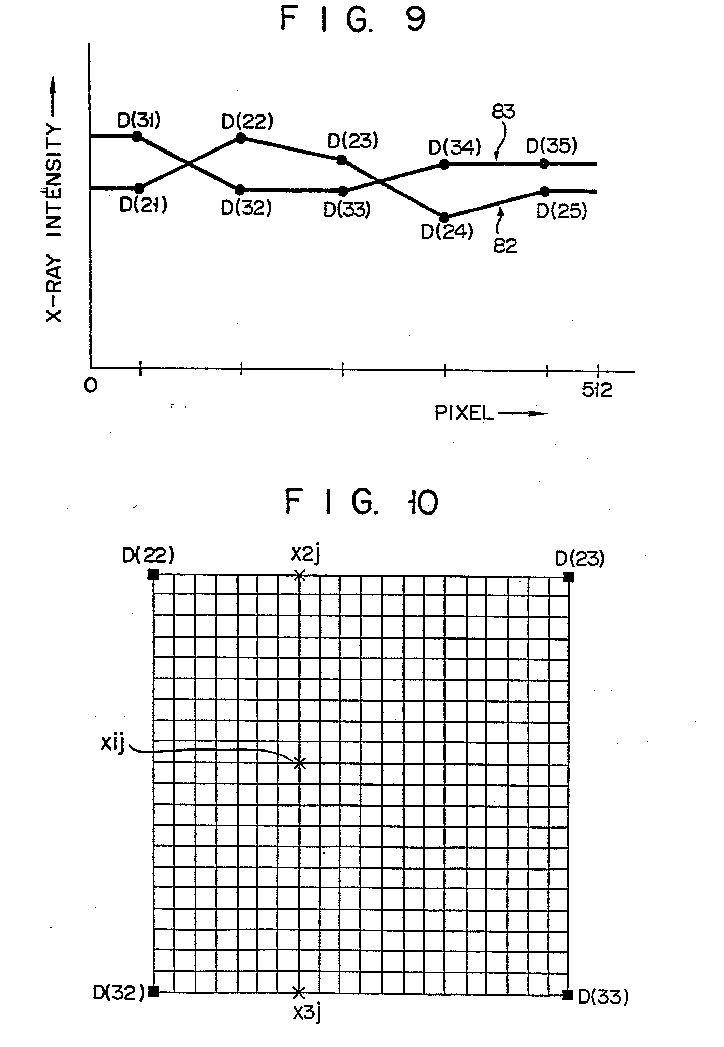

Fig. 8 shows an image pattern caused by the X-ray shield member 50, which is obtained by the X-ray detector 22, e.g., the image intensifier tube (not shown) and stored in the first memory 24. In this image pattern, it is assumed that the shadow indicated by numbers $P_{11}$, $P_{12}$, ..., $P_{55}$ corresponds to the images defined by the lead pieces 56 of the X-ray shield member 50. A line 82 parallel to the abscessa passes through the shadow $P_{21}$, $P_{22}$, $P_{23}$, $P_{24}$ and $P_{25}$. A line 83 parallel to the abscessa passes through the shadow $P_{31}$, $P_{32}$, $P_{33}$, $P_{34}$ and $P_{35}$. Fig. 9 shows intensity distributions of the scattered X-ray components along the lines 82 and 83, which have been obtained by utilizing the linear interpolation method. In Fig. 9, the abscessa represents pixel numbers, and the ordinate indicates X-ray intensities. Further, a value Dij (ij are for example 31 and 25) represents the intensity of the scattered X-ray component of the above-described images Pij (ij are for instance 11 and 55). This intensity is obtained from the actual measurement as previously described with respect to Fig. 7. To the contrary, the intensities between two adjacent values, e.g., $D_{22}$ and $D_{23}$ are calculated by utilizing the following linear interpolation method.

A description will now be made of an example of the linear interpolation with reference to Fig. 10.

Fig. 10 shows a region surrounded by the shadow $P_{22}$, $P_{23}$, $P_{32}$ and $P_{33}$ corresponding to the actual values $D_{22}$, $D_{23}$, $D_{32}$ and $D_{33}$. The following calculation on the linear interpolation method is directed to interpolation data for each of the pixels in the region.

It should be noted that in practice there are a great number of pixels other than the pixels as shown in Fig. 10.

A first linear interpolation is carried out for obtaining the interpolation data of the pixels along the line across the actual values $D_{22}$ and $D_{23}$. That is, the desired interpolation data of the arbitrary pixel $X_{2j}$ (i.e., the intensity of the scattered X-ray component thereof) can be calculated by the following equation:

$$d(X_{2j}) = \frac{D_{23} - D_{22}}{X_{23} - X_{22}} \cdot (X_{2j} - X_{22}) + D_{22} \quad \cdots \ (2),$$

where $X_{22}$ and $X_{23}$ denote the pixel numbers of the shadow $P_{22}$ and $P_{23}$ shown in Fig. 8.

As a result, the interpolation data for all of the pixels located between the actual values of the scattered X-ray components $D_{22}$ and $D_{23}$ can be obtained by the above equation (2).

Similarly, a second linear interpolation is done for the arbitrary pixel $X_{3j}$ along the abscessa passing through the actual values $D_{32}$ and $D_{33}$ based on the following equation:

$$d(X_{3j}) = \frac{D_{33} - D_{32}}{X_{33} - X_{32}} \cdot (X_{3j} - X_{32}) + D_{32} \quad \cdots \quad (3).$$

Consequently, the interpolation data (the scattered X-ray intensities) of all of the pixels located in the region defined by the actual values $D_{22}$, $D_{23}$, $D_{32}$ and $D_{33}$ can be easily obtained by employing two sets of the interpolation data that have been calculated from the equations 2 and 3.

That is to say, the interpolation data of the pixel $X_{ij}$ parallel to the ordinate is calculated based on two sets of the data $d(X_{2j})$ and $d(X_{3j})$ of the pixels $X_{2j}$ and $X_{3j}$ as follows:

$$d(X_{ij}) = \frac{d(X_{3j}) - d(X_{2j})}{X_{3j} - X_{2j}} \cdot (X_{ij} - X_{2j}) + d(X_{2j}) \quad \cdots \cdots \quad (4).$$

It is understood that the other interpolation data of all pixels surrounded by the four corner shadow $P_{11}$, $P_{15}$, $P_{51}$ and $P_{55}$ can be calculated by introducing the above calculation method. However, such an interpolation calculation cannot be directly applied to the remaining pixels located outside the region defined by the corner shadow $P_{11}$, $P_{15}$, $P_{51}$ and $P_{55}$. Accordingly, the interpolation data for the pixels located on the lines which intersect the above shadow $P_{11}$, $P_{15}$, $P_{51}$ and $P_{55}$ may be used as the desired interpolation data (as indicated by, for example, a horizontal line connecting the actual value $D_{21}$ and the ordinate). As a result, the entire intensity amounts of the scattered X-rays can be calculated for the storage region of the first memory 24 (512 × 512 pixel numbers), whereby the desired spatial distribution of the X-ray intensity can be obtained.

As previously described, the resultant intensity amounts are stored as the first X-ray transmission data in the first memory 24.

In accordance with the above-described embodiment, first, the scattered X-ray image data is obtained by interposing the X-ray shield member 50 between the X-ray source 30 and the X-ray detector 22 within the X-ray projection area 38. The data representing the scattered X-ray components is then calculated from the first X-ray transmission data by utilizing the linear interpolation method. Subsequently, the second X-ray transmission data is obtained by removing the X-ray shield member 50 from the X-ray projection area 38. Then the subtraction is effected between the data representing the scattered X-ray components and the second X-ray transmission data so as to obtain the X-ray image data having the better image qualities. In general, since a plurality of second X-ray transmission data is needed for the medical purposes, the above calculation of the linear interpolation method can be completed during the second data acquisition. As a result, the advantage of this embodiment is that the acquisition of the X-ray image data without any adverse effect by the scattered X-ray components can be achieved.

While the invention has been described in terms of certain preferred embodiments, and exemplified with respect thereto, those skilled in the art will readily appreciate that various modifications changes, omissions, and substitutions may be made without departing from the spirit of the invention.

For example, in the previous embodiment the first X-ray transmission data was obtained by inserting the X-ray shield member into the X-ray projection area and thereafter, the second X-ray transmission data was obtained by removing it from the X-ray projection area. It is also possible to change the sequence of the X-ray data acquisition.

Further, the X-ray shield member may be interposed between the patient 34 and the X-ray source 30.

The linear interpolation method may be, of course, substituted by, for example, utilizing the spline function.

Claims:

1.  An X-ray diagnostic apparatus (100)
characterized by comprising:

an X-ray source (30) for successively generating
X-rays;

means (22) for detecting an X-ray image of an
object (34) under examination by projecting the X-rays
from the X-ray source (30) toward the object (34),
and for converting the detected image into X-ray
transmission signals;

an analogue-to-digital converter (23) for
converting the X-ray transmission signals into
corresponding digital transmission data;

an X-ray shield member (50) having a plurality of
X-ray shield materials (56), for partially blocking the
penetration of the X-rays over an X-ray projection area
(38) defined by projecting the X-ray from the X-ray
source (30) to the X-ray detection means (22) through
the object (34);

first memory means (24) for storing at least first
X-ray transmission data acquired during a first X-ray
projection period under the condition that the X-ray
shield member (50) is inserted into the X-ray projection
area (38);

first arithmetic operation means (26) for obtaining
data representing an intensity distribution (Isc) of
scattered X-ray components, based upon the first
transmission data, over the entire X-ray projection area
by way of an interpolation method;

second memory means (25) for storing at least
second transmission data acquired during a second X-ray
projection period under the condition that the X-ray
shield member (50) is removed from the X-ray projection
area (38); and

second arithmetic operation means (25) for
subtracting said data representing the intensity distribution of scattered X-ray components from the second transmission data, thereby obtaining X-ray transmission data having no adverse influence by the scattered X-ray components.

2. An apparatus (100) as claimed in Claim 1, characterized in that the X-ray shield member (50) is constructed by a synthetic resin film (58) and a plurality of lead pieces (56) positioned in a matrix.

3. An apparatus (100) as claimed in Claim 1, characterized by further comprising drive means (54) for interruptedly inserting the X-ray shield member (50) into the X-ray projection area (38) during the first and second X-ray projection periods.

4. An apparatus (100) as claimed in Claim 3, characterized in that the drive means is constructed by a slide device (54) for sliding the X-ray shield member (50) along a longitudinal axis of a couch (30) on which the object (34) lies down.

5. An apparatus (100) as claimed in Claim 1, characterized in that the X-ray shield member (50) is positioned adjacent to the X-ray source (30) within the X-ray projection area (38).

6. An apparatus (100) as claimed in Claim 1, characterized in that both the first and second arithmetic operation means (26; 27) are united into a single arithmetic operation device.

7. An apparatus (100) as claimed in Claim 1, characterized in that both the first and second arithmetic operation means (26; 27) are united into a single arithmetic operation device, and both the first and second memory means (24; 25) are merged into a single memory device.

8. An apparatus (100) as claimed in Claim 1, characterized in that the first arithmetic operation means (26) employs a linear interpolation method for obtaining the data representing the intensity distribution of scattered X-ray components.

9.  An apparatus (100) as claimed in Claim 1, characterized in that the first arithmetic operation to obtain the data representing the intensity distribution of scattered X-ray components is effected prior over the second arithmetic operation to subtract the data representing the intensity distribution of scattered X-ray components from the second transmission data, whereby a read-time display of the X-ray transmission image can be realized.

# F I G. 1

# F I G. 2

F I G. 3A

F I G. 3B

F I G. 3C

F I G. 4

# F I G. 5

# F I G. 6

# F I G. 7

# F I G. 8

0142864

# F I G. 9

# F I G. 10